# EUROPEAN PATENT APPLICATION

(11) **EP 4 813 964 A1**
(43) Date of publication of application: **30.09.2026**
(21) Application number: 26730389.9
(22) Date of filing: 28.01.2026
(51) Int. Cl.: C07C 21/22, C07C 17/38

(54) **COMPOSITION CONTAINING HALOGENATED ALKYNE COMPOUND**

(30) Priority: 31.01.2025 JP 2025015457; 31.01.2025 JP 2025015458; 31.01.2025 JP 2025015461; 31.01.2025 JP 2025015462; 31.01.2025 JP 2025015465
(71) Applicant: DAIKIN INDUSTRIES, LTD., Osaka-shi, Osaka 530-0001 (JP)
(72) Inventor: ETO, Yusuke, Osaka-shi, Osaka 530-0001 (JP); NAKAMURA, Shingo, Osaka-shi, Osaka 530-0001 (JP); KUROKI, Yoshichika, Osaka-shi, Osaka 530-0001 (JP); SUGIYAMA, Akinari, Osaka-shi, Osaka 530-0001 (JP)
(74) Representative: Hoffmann Eitle
(86) International application number: PCT/JP2026/002927
(87) International publication number: WO 2026/164174

(57) **Abstract**

An object of the present disclosure is to newly provide a composition containing a halogenated alkyne compound. The composition contains a halogenated alkyne compound.

## Description

### Technical Field

The present disclosure relates to a composition containing a halogenated alkyne compound.

### Background Art

PTL 1 discloses a method for producing a perfluoroalkyne compound, comprising reacting a perfluoroalkadiene compound in the presence of a catalyst to obtain a perfluoroalkyne compound; a composition comprising a perfluoroalkyne compound and a perfluorocycloalkene compound; a catalyst for use in reacting a perfluoroalkadiene compound to obtain a perfluoroalkyne compound; and a method for producing a catalyst for use in reacting a perfluoroalkadiene compound to obtain a perfluoroalkyne compound.

PTL 2 discloses a method for producing a halogenated butene compound, comprising subjecting a halogenated butane compound to a dehydrofluorination reaction; a method for producing a halogenated butyne compound, comprising subjecting a halogenated butene compound to a dehydrohalogenation reaction; and a method for producing a halogenated butyne compound, comprising subjecting a halogenated butane compound to a dehydrofluorination reaction to produce a halogenated butene compound and then subjecting the halogenated butene compound to a dehydrohalogenation reaction to produce a halogenated butyne compound.

PTL 3 discloses a method for producing a perfluorocarbon compound, comprising reacting a halogenated hydrocarbon compound in an organic solvent in the presence of an iodine-containing inorganic material and zinc or a zinc alloy to obtain a perfluorocarbon compound; and a method for producing a perfluoroalkyne compound, comprising reacting a halogenated alkene compound in at least one nitrogen-containing polar organic solvent selected from the group consisting of N,N-dimethylacetamide, N,N-diisopropylformamide, N-methyl-2-pyrrolidone, 1,3-dimethyl-3,4,5,6-tetrahydropyrimidinone, hexamethylphosphoric triamide, amine compounds, pyridine compounds, and quinoline compounds in the presence of zinc or a zinc alloy to obtain a perfluoroalkyne compound.

### Citation List

### Patent Literature

PTL 1: WO2020/075728A1
PTL 2: WO2020/171011A1
PTL 3: WO2020/204146A1

### Summary of Invention

### Technical Problem

An object of the present disclosure is to newly provide a composition containing a halogenated alkyne compound.

### Solution to Problem

The present disclosure includes the following subject matter.

### Item 1.

A composition comprising a halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
   (2-1) a halogenated ethylene compound represented by formula (2): CF₂=CFX, wherein X represents a hydrogen atom, a chlorine atom, a bromine atom, or an iodine atom,
   (2-2) oxygen,
   (2-3) water,
   (2-4) hydrogen fluoride, and
   (2-5) hydrogen chloride,
wherein
in (2), based on the total amount of the composition,
when the composition contains (2-1) the halogenated ethylene compound represented by formula (2), the content of the halogenated ethylene compound represented by formula (2) is 1 vol ppm or more and 100 vol ppm or less,
when the composition contains (2-2) the oxygen, the content of the oxygen is 0.1 vol ppm or more and 100 vol ppm or less,
when the composition contains (2-3) the water, the content of the water is 0.1 vol ppm or more and 200 vol ppm or less,
when the composition contains (2-4) the hydrogen fluoride, the content of the hydrogen fluoride is 0.1 vol ppm or more and 10 vol ppm or less, or
when the composition contains (2-5) the hydrogen chloride, the content of the hydrogen chloride is 0.1 vol ppm or more and 10 vol ppm or less.

### Item 2.

The composition according to Item 1, wherein the content of (1) the halogenated alkyne compound represented by formula (1) is 95 vol% to 99.9999 vol% based on the total amount of the composition.

### Item 3.

A method for storing a composition containing a halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
   (2-1) a halogenated ethylene compound represented by formula (2): CF₂=CFX, wherein X represents a hydrogen atom, a chlorine atom, a bromine atom, or an iodine atom,
   (2-2) oxygen,
   (2-3) water,
   (2-4) hydrogen fluoride, and
   (2-5) hydrogen chloride,
the method comprising, in (2), based on the total amount of the composition,
adjusting the content of (2-1) the halogenated ethylene compound represented by formula (2) to 1 vol ppm or more and 100 vol ppm or less when the composition contains the halogenated ethylene compound represented by formula (2),
adjusting the content of (2-2) the oxygen to 0.1 vol ppm or more and 100 vol ppm or less when the composition contains the oxygen,
adjusting the content of (2-3) the water to 0.1 vol ppm or more and 200 vol ppm or less when the composition contains the water,
adjusting the content of (2-4) the hydrogen fluoride to 0.1 vol ppm or more and 10 vol ppm or less when the composition contains the hydrogen fluoride, or
adjusting the content of (2-5) the hydrogen chloride to 0.1 vol ppm or more and 10 vol ppm or less when the composition contains the hydrogen chloride.

### Item 4.

The method according to Item 3, comprising adjusting the content of (1) the halogenated alkyne compound represented by formula (1) to 95 vol% to 99.9999 vol% based on the total amount of the composition.

### Item 5.

A method for suppressing a decrease in purity of a halogenated alkyne compound in a composition containing the halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
   (2-1) a halogenated ethylene compound represented by formula (2): CF₂=CFX, wherein X represents a hydrogen atom, a chlorine atom, a bromine atom, or an iodine atom,
   (2-2) oxygen,
   (2-3) water,
   (2-4) hydrogen fluoride, and
   (2-5) hydrogen chloride,
the method comprising, in (2), based on the total amount of the composition,
adjusting the content of (2-1) the halogenated ethylene compound represented by formula (2) to 1 vol ppm or more and 100 vol ppm or less when the composition contains the halogenated ethylene compound represented by formula (2),
adjusting the content of (2-2) the oxygen to 0.1 vol ppm or more and 100 vol ppm or less when the composition contains the oxygen,
adjusting the content of (2-3) the water to 0.1 vol ppm or more and 200 vol ppm or less when the composition contains the water,
adjusting the content of (2-4) the hydrogen fluoride to 0.1 vol ppm or more and 10 vol ppm or less when the composition contains the hydrogen fluoride, or
adjusting the content of (2-5) the hydrogen chloride to 0.1 vol ppm or more and 10 vol ppm or less when the composition contains the hydrogen chloride.

### Item 6.

The method according to Item 5, comprising adjusting the content of (1) the halogenated alkyne compound represented by formula (1) to 95 vol% to 99.9999 vol% based on the total amount of the composition.

The present disclosure discloses a method for stably transporting a composition containing a halogenated alkyne compound and at least one component selected from the group consisting of a halogenated ethylene compound, oxygen, water, hydrogen fluoride, and hydrogen chloride. Due to the adjustment of the content (concentration) of the at least one component selected from the group consisting of a halogenated ethylene compound, oxygen, water, hydrogen fluoride, and hydrogen chloride in the composition containing a halogenated alkyne compound, the present disclosure can suppress the polymerization or decomposition of the halogenated alkyne compound, thus suppressing the decrease in purity of the halogenated alkyne compound, and maintaining the storage stability of the composition containing the halogenated alkyne compound, during the transportation of the composition containing the halogenated alkyne compound in a container such as a cylinder.

The composition containing a halogenated alkyne compound of the present disclosure contains at least one component selected from the group consisting of a halogenated ethylene compound, oxygen, water, hydrogen fluoride, and hydrogen chloride, has improved storage stability, and suppresses polymerization or decomposition of the halogenated alkyne compound even under severe conditions during marine transportation in containers (e.g. temperature rise during transportation); thus, it is possible to stably transport the composition containing the halogenated alkyne compound.

### Advantageous Effects of Invention

The present disclosure newly provides a composition containing a halogenated alkyne compound.

### Description of Embodiments

In the present specification, the terms "comprise," "contain," and "include" encompass the concepts of comprising, consisting essentially of, and consisting of. In the present specification, a numerical range indicated by "A to B" means A or more and B or less. In the present specification, expressions such as "part(s)" and "%" denote part(s) by mass, part(s) by weight, mass percent (mass%), or weight percent (wt%).

### [1] Composition Containing Halogenated Alkyne Compound

An olefin, oxygen (O₂), water, hydrogen fluoride (HF), or hydrogen chloride (HCl) present in a halogenated alkyne compound (e.g., butyne) may lead to polymerization (dimerization etc.) of the halogenated alkyne compound or olefin, reduced purity of the halogenated alkyne compound, or blockage within a container.

The composition containing a halogenated alkyne compound of the present disclosure suppresses a decrease in purity even during storage due to the adjustment of the concentration of at least one component selected from the group consisting of a halogenated ethylene compound (olefin), oxygen, water, hydrogen fluoride, and hydrogen chloride.

The composition containing a halogenated alkyne compound of the present disclosure suppresses a decrease in purity of the halogenated alkyne compound even during storage and suppresses blockage within a container.

(1-1) The composition containing a halogenated alkyne compound of the present disclosure contains
(1) a halogenated alkyne compound represented by formula (1): R¹-C≡C-R², and
(2) a halogenated ethylene compound represented by formula (2): CF₂=CFX,
wherein the content of (2) the halogenated ethylene compound represented by formula (2) is 1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

In formula (2), X represents a hydrogen atom, a chlorine atom, a bromine atom, or an iodine atom.

In the composition containing a halogenated alkyne compound of the present disclosure, the content of (1) the halogenated alkyne compound represented by formula (1) is preferably 95 vol% to 99.9999 vol% based on the total amount of the composition.

(1-2) The composition containing a halogenated alkyne compound of the present disclosure contains
(1) a halogenated alkyne compound represented by formula (1): R¹-C≡C-R², and
(2) oxygen,
wherein the content of (2) the oxygen is 0.1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

In the composition containing a halogenated alkyne compound of the present disclosure, the content of (1) the halogenated alkyne compound represented by formula (1) is preferably 95 vol% to 99.9999 vol% based on the total amount of the composition.

(1-3) The composition containing a halogenated alkyne compound of the present disclosure contains
(1) a halogenated alkyne compound represented by formula (1): R¹-C≡C-R², and
(2) water,
wherein the content of (2) the water is 0.1 vol ppm or more and 200 vol ppm or less based on the total amount of the composition.

In formula (1), R¹ represents a perfluoroalkyl group.

In formula (1), R² represents a perfluoroalkyl group, a hydrogen atom, or a fluorine atom.

In the composition containing a halogenated alkyne compound of the present disclosure, the content of (1) the halogenated alkyne compound represented by formula (1) is preferably 95 vol% to 99.9999 vol% based on the total amount of the composition.

(1-4) The composition containing a halogenated alkyne compound of the present disclosure exhibits a suppressed decrease in purity of the halogenated alkyne compound during storage and suppresses blockage within a container.

The composition containing a halogenated alkyne compound of the present disclosure contains
(1) a halogenated alkyne compound represented by formula (1): R¹-C≡C-R², and
(2) hydrogen fluoride,
wherein the content of (2) the hydrogen fluoride is 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

In the composition containing a halogenated alkyne compound of the present disclosure, the content of (1) the halogenated alkyne compound represented by formula (1) is preferably 95 vol% to 99.9999 vol% based on the total amount of the composition.

(1-5) The composition containing a halogenated alkyne compound of the present disclosure contains
(1) a halogenated alkyne compound represented by formula (1): R¹-C≡C-R², and
(2) hydrogen chloride,
wherein the content of (2) the hydrogen chloride is 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

In the composition containing a halogenated alkyne compound of the present disclosure, the content of the (1) halogenated alkyne compound represented by formula (1) is preferably 95 vol% to 99.9999 vol% based on the total amount of the composition.

The present disclosure discloses a method for stably transporting a composition containing a halogenated alkyne compound and at least one component selected from the group consisting of a halogenated ethylene compound, oxygen, water, hydrogen fluoride, and hydrogen chloride. Due to the adjustment of the content (concentration) of the at least one component selected from the group consisting of a halogenated ethylene compound, oxygen, water, hydrogen fluoride, and hydrogen chloride in the composition containing a halogenated alkyne compound, the present disclosure can suppress the polymerization or decomposition of the halogenated alkyne compound, thus suppressing the decrease in purity of the halogenated alkyne compound, and maintaining the storage stability of the composition containing the halogenated alkyne compound, during the transportation of the composition containing the halogenated alkyne compound in a container such as a cylinder.

The composition containing a halogenated alkyne compound of the present disclosure contains at least one component selected from the group consisting of a halogenated ethylene compound, oxygen, water, hydrogen fluoride, and hydrogen chloride, has improved storage stability, and suppresses polymerization or decomposition of the halogenated alkyne compound even under severe conditions during marine transportation in containers (e.g., temperature rise during transportation); thus, it is possible to stably transport the composition containing the halogenated alkyne compound.

### (1) Halogenated Alkyne Compound

The composition containing a halogenated alkyne compound of the present disclosure contains, as a first component, (1) a halogenated alkyne compound represented by formula (1): R¹-C≡C-R².

In formula (1), R¹ represents a perfluoroalkyl group.

In formula (1), R² represents a perfluoroalkyl group, a hydrogen atom (H), or a fluorine atom (F).

A perfluoroalkyl group is an alkyl group in which all hydrogen atoms are replaced by fluorine atoms.

The perfluoroalkyl group is preferably a C₁-C₈, more preferably C₁-C₆, even more preferably C₁-C₄, particularly preferably C₁-C₃, and most preferably C₁-C₂ (CF₃-, C₂F₅-) perfluoroalkyl group.

The perfluoroalkyl group is preferably a linear or branched perfluoroalkyl group. The perfluoroalkyl group is preferably a trifluoromethyl group (CF₃-), a pentafluoroethyl group (C₂F₅-: CF₃-CF₂-), a (linear) heptafluoropropyl group (C₃F₇-: CF₃-CF₂-CF₂-), a (branched) heptafluoroisopropyl group (C₃F₇-: CF₃-CF(CF₃)-), a (linear) pentafluoropropenyl group (C₃F₅-: CF₃-CF=CF-), a (branched) pentafluoroisopropenyl group (C₃F₅-: CF₂=C(CF₃)-), a (linear) nonafluorobutyl group (C₄F₉-: CF₃-CF₂-CF₂-CF₂-), a (branched) nonafluoro-sec-butyl group (C₄F₉-: CF₃-CF₂-CF(CF₃)-), a (branched) nonafluoro-tert-butyl group (C₄F₉-: CF₃-C(CF₃)₂-), or the like.
(1) The halogenated alkyne compound is preferably a C₃-C₈, more preferably C₃-C₆, even more preferably C₃-C₅, and particularly preferably C₄ halogenated alkyne compound.
(1) The halogenated alkyne compound is preferably 1,1,1,4,4,4-hexafluoro-2-butyne (CF₃C≡CCF₃).

The composition containing a halogenated alkyne compound may contain as (1) the halogenated alkyne compound the halogenated alkyne compound described above singly or in a combination (blend) of two or more kinds.

In the composition containing a halogenated alkyne compound, the content of (1) the halogenated alkyne compound (which is one kind alone, or the total amount when the halogenated alkyne compound includes two or more kinds) is preferably 95 vol% to 99.9999 vol% based on the total amount of the composition.

The composition containing a halogenated alkyne compound contains (1) the halogenated alkyne compound (first component) as a main component.

From the viewpoint of suppressing the formation of polymers (such as dimers) or decomposed products of a halogenated alkyne compound even during storage of the composition containing the halogenated alkyne compound, the content of (1) the halogenated alkyne compound in the composition is preferably, in order, 95 vol% to 99.9999 vol%, 96 vol% to 99.9997 vol%, 97 vol% to 99.9995 vol%; more preferably 95 vol% to 99.999 vol%, 97 vol% to 99.995 vol%, 97 vol% to 99.9995 vol%; even more preferably, in order, 98 vol% to 99.9990 vol%, 98 vol% to 99.990 vol%; and particularly preferably, in order, 99 vol% to 99.9985 vol%, 99 vol% to 99.985 vol%, based on the total amount of the composition.

### (2) At Least One Component Selected from the Group Consisting of Halogenated Ethylene Compound, Oxygen, Water, Hydrogen Fluoride, and Hydrogen Chloride

### (2-1) Halogenated Ethylene Compound

The composition containing a halogenated alkyne compound of the present disclosure contains, as a second component, (2) a halogenated ethylene compound represented by formula (2): CF₂=CFX.

In formula (2), X represents a hydrogen atom (H), a chlorine atom (Cl), a bromine atom (Br), or an iodine atom (I).

In the composition containing a halogenated alkyne compound, the content of (2) the halogenated ethylene compound (which is one kind, or the total amount of the halogenated ethylene compound when the halogenated ethylene compound includes two or more kinds) is 1 ppm by volume (vol ppm) or more and 100 ppm by volume (vol ppm) or less based on the total amount of the composition.

From the viewpoint of suppressing the formation of polymers (such as dimers) or decomposed products of a halogenated alkyne compound even during storage of the composition containing the halogenated alkyne compound, the halogenated ethylene compound is more preferably at least one halogenated ethylene compound selected from the group consisting of chlorotrifluoroethylene (CTFE) and bromotrifluoroethylene (BTFE), and even more preferably chlorotrifluoroethylene (CTFE).

The composition containing a halogenated alkyne compound may contain (2) the halogenated ethylene compound singly or in a combination (blend) of two or more kinds.

In the composition containing a halogenated alkyne compound, the content of (2) the halogenated ethylene compound represented by formula (2) (which is one kind alone, or the total amount when the halogenated ethylene compound includes two or more kinds) is 1 ppm by volume (vol ppm) or more and 100 ppm by volume (vol ppm) or less, more preferably 2 vol ppm to 99 vol ppm, even more preferably 3 vol ppm to 98 vol ppm, and particularly preferably 4 vol ppm to 97 vol ppm, based on the total amount of the composition.

The composition containing a halogenated alkyne compound of the present disclosure can suppress the formation of polymers (such as dimers) or decomposed products of the halogenated alkyne compound even during storage due to adjustment of the concentration of the halogenated ethylene compound (olefin compound).

The composition containing a halogenated alkyne compound of the present disclosure exhibits a suppressed decrease in purity of the halogenated alkyne compound even during storage and can suppress blockage within a container during storage.

### (2-2) Oxygen

The composition containing a halogenated alkyne compound of the present disclosure contains (2) oxygen (O₂) as a second component.

In the composition containing a halogenated alkyne compound, the content of (2) the oxygen is 0.1 ppm by volume (vol ppm) or more and 100 ppm by volume (vol ppm) or less based on the total amount of the composition.

In the composition containing a halogenated alkyne compound, the content of (2) the oxygen is 0.1 ppm by volume (vol ppm) or more and 100 ppm by volume (vol ppm) or less, more preferably 0.3 vol ppm to 75 vol ppm, even more preferably 0.6 vol ppm to 50 vol ppm, and particularly preferably 1 vol ppm to 15 vol ppm, based on the total amount of the composition.

The composition containing a halogenated alkyne compound of the present disclosure can suppress the formation of polymers (such as dimers) or decomposed products of the halogenated alkyne compound even during storage due to adjustment of the concentration of oxygen.

The composition containing a halogenated alkyne compound of the present disclosure can exhibit a suppressed decrease in purity of the halogenated alkyne compound even during storage and can suppress blockage within a container during storage.

### (2-3) Water

The composition containing a halogenated alkyne compound of the present disclosure contains (2) water as a second component.

In the composition containing a halogenated alkyne compound, the content of (2) the water is 0.1 ppm by volume (vol ppm) or more and 200 ppm by volume (vol ppm) or less based on the total amount of the composition.

In the composition containing a halogenated alkyne compound, the content of (2) the water is 0.1 ppm by volume (vol ppm) or more and 200 ppm by volume (vol ppm) or less, more preferably 0.3 vol ppm to 170 vol ppm, even more preferably 0.5 vol ppm to 150 vol ppm, and particularly preferably 1 vol ppm to 100 vol ppm, based on the total amount of the composition.

The composition containing a halogenated alkyne compound of the present disclosure can suppress the formation of polymers (such as dimers) or decomposed products of the halogenated alkyne compound even during storage due to adjustment of the concentration of water.

The composition containing a halogenated alkyne compound of the present disclosure exhibits a suppressed decrease in purity of the halogenated alkyne compound during storage and can suppress blockage within a container during storage.

### (2-4) Hydrogen Fluoride

The composition containing a halogenated alkyne compound of the present disclosure contains (2) hydrogen fluoride (HF) as a second component.

In the composition containing a halogenated alkyne compound, the content of (2) the hydrogen fluoride is 0.1 ppm by volume (vol ppm) or more and 10 ppm by volume (vol ppm) or less based on the total amount of the composition.

In the composition containing a halogenated alkyne compound, the content of (2) the hydrogen fluoride is 0.1 ppm by volume (vol ppm) or more and 10 ppm by volume (vol ppm) or less, more preferably 0.3 vol ppm to 7.5 vol ppm, even more preferably 0.5 vol ppm to 6 vol ppm, and particularly preferably 1 vol ppm to 4 vol ppm, based on the total amount of the composition.

The composition containing a halogenated alkyne compound of the present disclosure can suppress the formation of polymers (such as dimers) or decomposed products of the halogenated alkyne compound even during storage due to adjustment of the concentration of hydrogen fluoride.

The composition containing a halogenated alkyne compound of the present disclosure exhibits a suppressed decrease in purity of the halogenated alkyne compound during storage and can suppress blockage within a container during storage.

### (2-5) Hydrogen Chloride

The composition containing a halogenated alkyne compound of the present disclosure contains (2) hydrogen chloride (HCl) as a second component.

In the composition containing a halogenated alkyne compound, the content of (2) the hydrogen chloride is 0.1 ppm by volume (vol ppm) or more and 10 ppm by volume (vol ppm) or less, based on the total amount of the composition.

In the composition containing a halogenated alkyne compound, the content of (2) the hydrogen chloride is 0.1 ppm by volume (vol ppm) or more and 10 ppm by volume (vol ppm) or less, more preferably 0.3 vol ppm to 7.5 vol ppm, even more preferably 0.5 vol ppm to 6 vol ppm, and particularly preferably 1 vol ppm to 4 vol ppm, based on the total amount of the composition.

The composition containing a halogenated alkyne compound of the present disclosure can suppress the formation of polymers (such as dimers) or decomposed products of the halogenated alkyne compound even during storage due to adjustment of the concentration of hydrogen chloride.

The composition containing a halogenated alkyne compound of the present disclosure exhibits a suppressed decrease in purity of the halogenated alkyne compound even during storage and can suppress blockage within a container during storage.

### [2] Method for Storing Composition Containing Halogenated Alkyne Compound

In the method for storing a composition containing a halogenated alkyne compound of the present disclosure, (2-1a) the composition contains
(1) a halogenated alkyne compound represented by formula (1): R¹-C≡C-R², and
(2) a halogenated ethylene compound represented by formula (2): CF₂=CFX, and
the method comprises adjusting the content of (2) the halogenated ethylene compound represented by formula (2) to 1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

In formula (2), X represents a hydrogen atom, a chlorine atom, a bromine atom, or an iodine atom.

The method for storing a composition containing a halogenated alkyne compound of the present disclosure preferably comprises adjusting the content of (1) the halogenated alkyne compound represented by formula (1) to 95 vol% to 99.9999 vol% based on the total amount of the composition.

In the method for suppressing a decrease in purity of a halogenated alkyne compound in the composition containing the halogenated alkyne compound of the present disclosure, (2-1b) the composition contains
(1) a halogenated alkyne compound represented by formula (1): R¹-C≡C-R², and
(2) a halogenated ethylene compound represented by formula (2): CF₂=CFX, and
the method comprises adjusting the content of (2) the halogenated ethylene compound represented by formula (2) to 1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

In formula (2), X represents a hydrogen atom, a chlorine atom, a bromine atom, or an iodine atom.

The method for suppressing a decrease in purity of a halogenated alkyne compound in the composition containing the halogenated alkyne compound of the present disclosure preferably comprises adjusting (1) the content of the halogenated alkyne compound represented by formula (1) to 95 vol% to 99.9999 vol% based on the total amount of the composition.

In the method for storing a composition containing a halogenated alkyne compound of the present disclosure, (2-2a) the composition contains
(1) a halogenated alkyne compound represented by formula (1): R¹-C≡C-R², and
(2) oxygen, and
the method comprises adjusting the content of (2) the oxygen to 0.1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

The method for storing a composition containing a halogenated alkyne compound of the present disclosure preferably comprises adjusting the content of (1) the halogenated alkyne compound represented by formula (1) to 95 vol% to 99.9999 vol% based on the total amount of the composition.

In the method for suppressing a decrease in purity of a halogenated alkyne compound in the composition containing the halogenated alkyne compound of the present disclosure, (2-2b) the composition contains
(1) a halogenated alkyne compound represented by formula (1): R¹-C≡C-R², and
(2) oxygen, and
the method comprises adjusting the content of (2) the oxygen to 0.1 vol ppm or more and 100 vol ppm or less based on the total amount of the composition.

In formula (1), R¹ represents a perfluoroalkyl group.

In formula (1), R² represents a perfluoroalkyl group, a hydrogen atom, or a fluorine atom.

The method for suppressing a decrease in purity of a halogenated alkyne compound in the composition containing the halogenated alkyne compound of the present disclosure preferably comprises adjusting (1) the halogenated alkyne compound represented by formula (1) to 95 vol% to 99.9999 vol% based on the total amount of the composition.

In the method for storing a composition containing a halogenated alkyne compound of the present disclosure, (2-3a) the composition contains
(1) a halogenated alkyne compound represented by formula (1): R¹-C≡C-R², and
(2) water, and
the method comprises adjusting the content of (2) the water to 0.1 vol ppm or more and 200 vol ppm or less based on the total amount of the composition.

In formula (1), R¹ represents a perfluoroalkyl group.

In formula (1), R² represents a perfluoroalkyl group, a hydrogen atom, or a fluorine atom.

The method for storing a composition containing a halogenated alkyne compound of the present disclosure preferably comprises adjusting the content of the (1) halogenated alkyne compound represented by formula (1) to 95 vol% to 99.9999 vol% based on the total amount of the composition.

In the method for suppressing a decrease in purity of a halogenated alkyne compound in the composition containing the halogenated alkyne compound of the present disclosure, (2-3b) the composition contains
(1) a halogenated alkyne compound represented by formula (1): R¹-C≡C-R², and
(2) water, and
the method comprises adjusting the content of (2) the water to 0.1 vol ppm or more and 200 vol ppm or less based on the total amount of the composition.

In formula (1), R¹ represents a perfluoroalkyl group.

In formula (1), R² represents a perfluoroalkyl group, a hydrogen atom, or a fluorine atom.

The method for suppressing a decrease in purity of a halogenated alkyne compound in the composition containing a halogenated alkyne compound of the present disclosure preferably comprises adjusting the content of (1) the halogenated alkyne compound represented by formula (1) to 95 vol% to 99.9999 vol% based on the total amount of the composition.

In the method for storing a composition containing a halogenated alkyne compound of the present disclosure, (2-4a) the composition contains
(1) a halogenated alkyne compound represented by formula (1): R¹-C≡C-R², and
(2) hydrogen fluoride, and
the method comprises adjusting the content of (2) the hydrogen fluoride to 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

The method for storing a composition containing a halogenated alkyne compound of the present disclosure preferably comprises adjusting the content of (1) the halogenated alkyne compound represented by formula (1) to 95 vol% to 99.9999 vol% based on the total amount of the composition.

In the method for suppressing a decrease in purity of a halogenated alkyne compound in the composition containing the halogenated alkyne compound of the present disclosure, (2-4b) the composition contains
(1) a halogenated alkyne compound represented by formula (1): R¹-C≡C-R², and
(2) hydrogen fluoride, and
the method comprises adjusting the content of the (2) hydrogen fluoride to 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

The method for suppressing a decrease in purity of a halogenated alkyne compound in the composition containing the halogenated alkyne compound of the present disclosure preferably comprises adjusting the content of (1) the halogenated alkyne compound represented by formula (1) to 95 vol% to 99.9999 vol% based on the total amount of the composition.

In the method for storing a composition containing a halogenated alkyne compound of the present disclosure, (2-5a) the composition contains (1)
a halogenated alkyne compound represented by formula (1): R¹-C≡C-R², and
(2) hydrogen chloride, and
the method comprises adjusting the content of (2) the hydrogen chloride to 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

The method for storing a composition containing a halogenated alkyne compound of the present disclosure preferably comprises adjusting the content of (1) the halogenated alkyne compound represented by formula (1) to 95 vol% to 99.9999 vol% based on the total amount of the composition.

In the method for suppressing a decrease in purity of a halogenated alkyne compound in the composition containing the halogenated alkyne compound of the present disclosure, (2-5b) the composition contains
(1) a halogenated alkyne compound represented by formula (1): R¹-C≡C-R², and
(2) hydrogen chloride, and
the method comprises adjusting the content of (2) the hydrogen chloride to 0.1 vol ppm or more and 10 vol ppm or less based on the total amount of the composition.

In formula (1), R¹ represents a perfluoroalkyl group.

In formula (1), R² represents a perfluoroalkyl group, a hydrogen atom, or a fluorine atom.

The method for suppressing a decrease in purity of a halogenated alkyne compound in the composition containing the halogenated alkyne compound of the present disclosure preferably comprises adjusting the content of (1) the halogenated alkyne compound represented by formula (1) to 95 vol% to 99.9999 vol% based on the total amount of the composition.

In the method for storing a composition containing a halogenated alkyne compound of the present disclosure and the method for suppressing a decrease in purity of a halogenated alkyne compound in the composition containing the halogenated alkyne compound, the details such as components and their content in the composition containing a halogenated alkyne compound described in section [1] Composition Containing Halogenated Alkyne Compound above apply.

The method for storing a composition containing a halogenated alkyne compound and the method for suppressing a decrease in purity of a halogenated alkyne compound in a composition containing the halogenated alkyne compound according to the present disclosure can suppress the formation of polymers (such as dimers) or decomposed products of the halogenated alkyne compound even during storage due to adjustment of the concentration of at least one component selected from the group consisting of a halogenated ethylene compound (olefin compound), oxygen (O₂), water, hydrogen fluoride (HF), and hydrogen chloride (HCl) in the composition containing the halogenated alkyne compound.

The method for storing a composition containing a halogenated alkyne compound and the method for suppressing a decrease in purity of a halogenated alkyne compound in a composition containing the halogenated alkyne compound according to the present disclosure allow the composition containing the halogenated alkyne compound to suppress a decrease in purity of the halogenated alkyne compound during storage and to suppress blockage within a container during storage.

The composition containing a halogenated alkyne compound of the present disclosure exhibits excellent storage stability when transported in a container, such as a cylinder.

The composition containing a halogenated alkyne compound of the present disclosure has improved storage stability and suppresses the polymerization or decomposition of the halogenated alkyne compound even under severe conditions during marine transportation in containers (e.g., temperature rise during transportation); thus, it is possible to stably transport the composition containing the halogenated alkyne compound.

### [3] Use of Composition Containing Halogenated Alkyne Compound

The composition containing a halogenated alkyne compound of the present disclosure is preferably used as an etching gas, a refrigerant, a heat transfer medium, a deposition gas, a building block for organic synthesis, or a cleaning gas.

The composition containing a halogenated alkyne compound of the present disclosure is preferably used as an etching gas, a refrigerant, a heat transfer medium, or the like for forming cutting-edge fine structures, such as semiconductors and liquid crystals. The composition is preferably used in a variety of applications, such as a deposition gas, a building block for organic synthesis, and a cleaning gas.

The "deposition gas" refers to a gas that deposits an etching-resistant polymer layer.

The "building block for organic synthesis" refers to a substance that can become a precursor of a compound that has a highly reactive skeleton. Reacting a composition containing (1) a halogenated alkyne compound represented by formula (1): R¹-C≡C-R² with a fluorine-containing organosilicon compound, such as CF₃Si(CH₃)₃, allows introduction of a fluoroalkyl group, such as a CF₃ group, converting the composition into a substance that can become a cleaning agent or a fluorine-containing pharmaceutical intermediate.

Etching can be performed on a substrate for semiconductor manufacturing by using, as an etching gas, the composition containing a halogenated alkyne compound of the present disclosure as a gas supplied for generating plasma.

A silicon-based material on which a silicon oxide film (SiO₂ film) or a silicon nitride film (SiN film) is formed can be etched with gas plasma of a composition containing a halogenated alkyne compound (etching gas). The silicon-based material to be etched is preferably a substrate for semiconductor manufacturing.

Hole etching on a silicon-based material having a silicon oxide film (SiO₂ film) or a silicon nitride film (SiN film) using gas plasma generated from a composition containing a halogenated alkyne compound results in excellent hole profile retention. The conditions for the etching method (in particular, a dry etching method) can be the same as those of a known method.

### Examples

The present invention is described below in detail with reference to Examples and Comparative Examples. The present invention is not limited to these Examples.

### [1] Storage Stability of Composition Containing Halogenated Alkyne Compound

### Example 1: Second Component (Halogenated Ethylene Compound) (1) Storage Stability Test

### Composition Containing Halogenated Alkyne Compound

(1) A halogenated alkyne compound represented by formula (1):

   R¹-C≡C-R²

   wherein R¹ and R² are as described in Table 1.
(2) A halogenated ethylene compound represented by formula (2):

   CF₂=CFX

   Chlorotrifluoroethylene (CTFE)
   Bromotrifluoroethylene (BTFE)

According to a commonly used method, (1) a halogenated alkyne compound and (2) a halogenated ethylene compound adjusted to a predetermined concentration were mixed to prepare a composition containing a halogenated alkyne compound.

The composition containing a halogenated alkyne compound of the Examples contained (2) the halogenated ethylene compound in an amount of 1 vol ppm or more and 100 vol ppm or less based on the total amount of the composition.

The concentration (vol ppm) of (2) the halogenated ethylene compound in the compositions containing a halogenated alkyne compound was analyzed by gas chromatography (GC analysis).

The compositions containing a halogenated alkyne compound were stored at a constant temperature (25°C). After a predetermined period of time (1 month) elapsed, the gas phase was extracted, and GC analysis (vol ppm) was performed for polymers and/or decomposed products derived from (1) the halogenated alkyne compound and/or (2) the halogenated ethylene compound.

**Table 1**

| Table 1 | | | | | | |
|---|---|---|---|---|---|---|
| | | | Initial Concentration | | 25°C, After 1 month | |
| | (1) Halogenated Alkyne Compound | | (2) Halogenated Ethylene Compound | | Polymer /Decomposed Product | Condition of Pipe Cross Section |
| | R¹ | R² | Kind | Concentration vol ppm | Concentration vol ppm | Pipe Blockage |
| Example 1-1 | CF₃- | CF₃ | CTFE | 97 | ND | None |
| Example 1-2 | CF₃- | CF₃ | CTFE | 1 | 0.7 | None |
| Example 1-3 | CF₃- | CF₃ | BTFE | 4 | ND | None |
| Comparative Example 1-4 | CF₃- | CF₃ | CTFE | 134 | 98 | Observed |
| Comparative Example 1-5 | CF₃- | CF₃ | CTFE | 0.4 | 13 | Observed |
| Example 1-6 | CF₃- | H | CTFE | **80** | ND | None |
| Example 1-7 | CF₃- | F | CTFE | **86** | ND | None |
| Example 1-8 | C₂F₅- | CF₃ | CTFE | **78** | ND | None |
| Example 1-9 | C₂F₅- | C₂F₅ | CTFE | **60** | ND | None |
| Example 1-10 | C₂F₅- | H | CTFE | **87** | ND | None |
| Example 1-11 | C₂F₅- | F | CTFE | **90** | ND | None |
| Example 1-12 | C₂F₅- | F | CTFE | **1.3** | 0.6 | None |
| Example 1-13 | C₂F₅- | F | BTFE | **2** | ND | None |
| Comparative Example 1-14 | C₂F₅- | F | CTFE | 120 | 100 | Observed |
| Comparative Example 1-15 | C₂F₅- | F | CTFE | 0.8 | 22 | Observed |

### (1) Halogenated Alkyne Compound: R¹-C≡C-R²

R¹ CF₃-: trifluoromethyl group, C₂F₅-: pentafluoroethyl group
R² CF₃-: trifluoromethyl group, C₂F₅-: pentafluoroethyl group
H: hydrogen atom, F: fluorine atom

### (2) Halogenated Ethylene Compound

CTFE: chlorotrifluoroethylene
BTFE: bromotrifluoroethylene
ND: Not Detected

### (2) Storage Stability Evaluation Results

Due to the adjustment of the concentration of the halogenated ethylene compound (olefin compound) to 1 vol ppm or more and 100 vol ppm or less based on the total amount of the composition, each composition containing the halogenated alkyne compound of the Examples was able to suppress the formation of polymers (such as dimers) or decomposed products of the halogenated alkyne compound within an allowable range even during storage (25°C, 1 month), as compared to the Comparative Examples in which that concentration fell outside this range.

The compositions containing the halogenated alkyne compound of the Examples suppressed a decrease in purity of the halogenated alkyne compound during storage and were able to suppress blockage within the container during storage.

### Example 2: Second Component (Oxygen (O₂))

### (1) Storage Stability Test

### Composition Containing Halogenated Alkyne Compound

(1) A halogenated alkyne compound represented by formula (1):

   R¹-C≡C-R²

   wherein
   R¹ CF₃-: trifluoromethyl group
   R² CF₃-: trifluoromethyl group
   1,1,1,4,4,4-hexafluoro-2-butyne (CF₃C≡CCF₃)

According to a commonly used method, (1) the halogenated alkyne compound and (2) oxygen adjusted to a predetermined concentration were mixed to prepare a composition containing a halogenated alkyne compound.

Each composition containing the halogenated alkyne compound of the Examples contained (2) oxygen in an amount of 0.1 vol ppm or more and 100 vol ppm or less based on the total amount of the composition.

The concentration of (2) the oxygen (vol ppm) in the compositions containing the halogenated alkyne compound was analyzed by gas chromatography (GC analysis).

The compositions containing the halogenated alkyne compound were stored at a constant temperature (25°C or 50°C). After a predetermined period of time (1 month) elapsed, the gas phase was extracted, and GC analysis (vol ppm) was performed for polymers and/or decomposed products derived from (1) the halogenated alkyne compound, and hydrogen fluoride (HF) generated during the formation of these polymers and/or decomposed products.

**Table 2**

| Table 2 | | | | | |
|---|---|---|---|---|---|
| | Storage | Initial | After Storage of 1 Month | | |
| | Temperature | Oxygen | HF | Polymer /Decomposed Product | Condition of Pipe Cross Section |
| | °C | Concentration vol ppm | Concentration vol ppm | Concentration vol ppm | Pipe Blockage |
| Comparative Example 2-1 | 25 | ND | ND | 10 | Observed |
| Example 2-2 | 25 | 1 | ND | ND | None |
| Example 2-3 | 25 | 5 | ND | ND | None |
| Example 2-4 | 25 | 10 | ND | ND | None |
| Reference Example 2-5 | 25 | 20 | >100 | 30 | None |
| Comparative Example 2-6 | 50 | ND | ND | 11 | Observed |
| Example 2-7 | 50 | 1 | ND | ND | None |
| Example 2-8 | 50 | 5 | ND | ND | None |
| Reference Example 2-9 | 50 | 10 | >100 | 21 | None |
| Reference Example 2-10 | 50 | 20 | >100 | 45 | None |

| | | | | | |
|---|---|---|---|---|---|
| ND: Not Detected | | | | | |

### (2) Storage Stability Evaluation Results

The compositions containing the halogenated alkyne compound of the Examples were able to suppress the formation of polymers (such as dimers) or decomposed products of the halogenated alkyne compound within an acceptable range during storage (at 25°C or 50°C, 1 month) due to the adjustment of the concentration of the oxygen to 0.1 vol ppm or more and 100 vol ppm or less.

The compositions containing the halogenated alkyne compound of the Examples suppressed a decrease in purity of the halogenated alkyne compound during storage and were able to suppress blockage within the container during storage.

### Example 3: Second Component (Water)

### (1) Storage Stability Test

### Composition Containing Halogenated Alkyne Compound

(1) A halogenated alkyne compound represented by formula (1):

   R¹-C≡C-R²

   R¹ CF₃-: trifluoromethyl group
   R² CF₃-: trifluoromethyl group
   1,1,1,4,4,4-hexafluoro-2-butyne (CF₃C≡CCF₃)

According to a commonly used method, (1) the halogenated alkyne compound and (2) water adjusted to a predetermined concentration were mixed to prepare a composition containing a halogenated alkyne compound.

Each composition containing the halogenated alkyne compound of the Examples contained (2) water in an amount of 0.1 vol ppm or more and 200 vol ppm or less based on the total amount of the composition.

The concentration of the (2) water (vol ppm) in the compositions containing the halogenated alkyne compound was analyzed by gas chromatography (GC analysis).

The compositions containing the halogenated alkyne compound were stored at a constant temperature (25°C, 40°C, or 60°C). After a predetermined period of time (1 month) elapsed, the gas phase was extracted, and GC analysis (vol ppm) was performed for polymers and/or decomposed products derived from (1) the halogenated alkyne compound, and hydrogen fluoride (HF) generated during the formation of these polymers and/or decomposed products.

**Table 3**

| Table 3 | | | | | |
|---|---|---|---|---|---|
| | Storage | Initial | After Storage of 1 Month | | |
| | Temperature | Water | HF | Polymer /Decomposed Product | Condition of Pipe Cross Section |
| | °C | Concentration vol ppm | Concentration vol ppm | Concentration vol ppm | Pipe Blockage |
| Comparative Example 3-1 | 25 | 0.05 | ND | 6 | Observed |
| Example 3-2 | 25 | **1** | ND | ND | None |
| Example 3-3 | 25 | **200** | ND | 0.6 | None |
| Example 3-4 | 40 | **0.5** | ND | 0.2 | None |
| Example 3-5 | 40 | **200** | ND | 0.7 | None |
| Comparative Example 3-6 | 40 | 1,000 | >100 | 27 | Observed |
| Example 3-7 | 60 | **0.5** | ND | 0.3 | None |
| Example 3-8 | 60 | **53** | ND | ND | None |
| Comparative Example 3-9 | 60 | 400 | >100 | 40 | Observed |

| | | | | | |
|---|---|---|---|---|---|
| ND: Not Detected | | | | | |

### (2) Storage Stability Evaluation Results

Due to the adjustment of the concentration of water to 0.1 vol ppm or more and 200 vol ppm or less based on the total amount of the individual composition, the compositions containing the halogenated alkyne compound of the Examples were able to suppress the formation of polymers (such as dimers) or decomposed products of the halogenated alkyne compound within an acceptable range during storage (at 25°C, 40°C, or 60°C, after 1 month), as compared to the Comparative Examples in which the concentration fell outside this range.

The compositions containing the halogenated alkyne compound of the Examples suppressed a decrease in purity of the halogenated alkyne compound during storage and were able to suppress blockage within the container during storage.

### Example 4: Second Component (Hydrogen Fluoride (HF))

### (1) Storage Stability Test

### Composition Containing Halogenated Alkyne Compound

(1) A halogenated alkyne compound represented by formula (1):

   R¹-C≡C-R²

   R¹ CF₃-: trifluoromethyl group
   R² CF₃-: trifluoromethyl group
   1,1,1,4,4,4-hexafluoro-2-butyne (CF₃C≡CCF₃)

According to a commonly used method, (1) the halogenated alkyne compound and (2) hydrogen fluoride adjusted to a predetermined concentration were mixed to prepare a composition containing a halogenated alkyne compound.

Each composition containing the halogenated alkyne compound of the Examples contained (2) hydrogen fluoride (HF) in an amount of 0.1 vol ppm or more and 10 vol ppm or less based on the total amount of the composition.

The concentration of (2) hydrogen fluoride (vol ppm) in the compositions containing the halogenated alkyne compound was analyzed by Fourier-transform infrared spectroscopy (FT-IR analysis).

The compositions containing the halogenated alkyne compound were stored at a constant temperature (25°C or 50°C). After a predetermined period of time (1 month) elapsed, the gas phase was extracted, and GC analysis (vol ppm) was performed for polymers and/or decomposed products derived from (1) the halogenated alkyne compound.

**Table 4**

| Table 4 | | | | |
|---|---|---|---|---|
| | Storage | Initial | After Storage of 1 Month | |
| | Temperature | HF | Polymer /Decomposed Product | Condition of Pipe Cross Section |
| | °C | Concentration vol ppm | Concentration vol ppm | Pipe Blockage |
| Comparative Example 4-1 | 25 | ND | 10 | Observed |
| Example 4-2 | 25 | **1** | ND | None |
| Example 4-3 | 25 | **5** | 0.6 | None |
| Example 4-4 | 25 | **10** | 0.7 | None |
| Comparative Example 4-5 | 25 | 20 | 28 | Observed |
| Comparative Example 4-6 | 50 | ND | 11 | Observed |
| Example 4-7 | 50 | **1** | ND | None |
| Example 4-8 | 50 | **5** | 0.7 | None |
| Example 4-9 | 50 | **10** | 0.8 | None |
| Comparative Example 4-10 | 50 | 20 | 52 | Observed |

| | | | | |
|---|---|---|---|---|
| ND: Not Detected | | | | |

### (2) Storage Stability Evaluation Results

Due to the adjustment of the concentration of hydrogen fluoride to 0.1 vol ppm or more and 10 vol ppm or less based on the total amount of the composition, each composition containing the halogenated alkyne compound of the Examples was able to suppress the formation of polymers (such as dimers) or decomposed products of the halogenated alkyne compound within an acceptable range during storage, as compared to the Comparative Examples in which the concentration fell outside this range.

The compositions containing the halogenated alkyne compound of the Examples suppressed a decrease in purity of the halogenated alkyne compound during storage (25°C or 50°C, after 1 month), and were able to suppress blockage within the container during storage.

### Example 5: Second Component (Hydrogen Chloride (HCl))

### (1) Storage Stability Test

### Composition Containing Halogenated Alkyne Compound

(1) A halogenated alkyne compound represented by formula (1):

   R¹-C≡C-R²

   R¹ CF₃-: trifluoromethyl group
   R² CF₃-: trifluoromethyl group
   1,1,1,4,4,4-hexafluoro-2-butyne (CF₃C≡CCF₃)

According to a commonly used method, (1) the halogenated alkyne compound and (2) hydrogen chloride adjusted to a predetermined concentration were mixed to prepare a composition containing the halogenated alkyne compound.

Each composition containing the halogenated alkyne compound of the Examples contained (2) hydrogen chloride (HCl) in an amount of 0.1 vol ppm or more and 10 vol ppm or less based on the total amount of the composition.

The concentration of (2) hydrogen chloride (vol ppm) in the composition containing the halogenated alkyne compound was analyzed by gas chromatography (GC analysis).

The compositions containing the halogenated alkyne compound were stored at a constant temperature (25°C or 50°C). After a predetermined period of time (1 month) elapsed, the gas phase was extracted, and GC analysis (vol ppm) was performed for polymers and/or decomposed products derived from (1) the halogenated alkyne compound.

**Table 5**

| Table 5 | | | | |
|---|---|---|---|---|
| | Storage | Initial | After Storage of 1 Month | |
| | Temperature | HCl | Polymer /Decomposed Product | Condition of Pipe Cross Section |
| | °C | Concentration vol ppm | Concentration vol ppm | Pipe Blockage |
| Comparative Example 5-1 | 25 | ND | 10 | Observed |
| Example 5-2 | 25 | **1** | ND | None |
| Example 5-3 | 25 | **5** | 0.2 | None |
| Example 5-4 | 25 | **10** | 0.5 | None |
| Comparative Example 5-5 | 25 | 20 | 18 | Observed |
| Comparative Example 5-6 | 50 | ND | 11 | Observed |
| Example 5-7 | 50 | **1** | ND | None |
| Example 5-8 | 50 | **5** | 0.3 | None |
| Example 5-9 | 50 | **10** | 0.6 | None |
| Comparative Example 5-10 | 50 | 20 | 27 | Observed |

| | | | | |
|---|---|---|---|---|
| ND: Not Detected | | | | |

### (2) Storage Stability Evaluation Results

Due to the adjustment of the concentration of hydrogen chloride to 0.1 vol ppm or more and 10 vol ppm or less based on the total amount of the composition, each composition containing the halogenated alkyne compound of the Examples was able to suppress the formation of polymers (such as dimers) or decomposed products of the halogenated alkyne compound within an acceptable range during storage (at 25°C or 50°C, 1 month), as compared to the Comparative Examples in which the concentration fell outside this range.

The compositions containing the halogenated alkyne compound of the Examples suppressed a decrease in purity of the halogenated alkyne compound during storage (25°C or 50°C, after 1 month), and were able to suppress blockage within the container during storage.

### [2] Industrial Applicability

The composition containing a halogenated alkyne compound of the present disclosure can suppress the formation of polymers (such as dimers) or decomposed products of the halogenated alkyne compound even during storage by adjusting the concentration of at least one component selected from the group consisting of a halogenated ethylene compound (olefin compound), oxygen, water, hydrogen fluoride, and hydrogen chloride.

The composition containing a halogenated alkyne compound of the present disclosure exhibits a suppressed decrease in purity of the halogenated alkyne compound during storage and can suppress blockage within a container during storage.

Due to the adjustment of the content (concentration) of at least one component selected from the group consisting of a halogenated ethylene compound, oxygen, water, hydrogen fluoride, and hydrogen chloride in the composition containing a halogenated alkyne compound according to the present disclosure, it becomes possible to suppress the polymerization or decomposition of the halogenated alkyne compound, thus suppressing the decrease in purity of the halogenated alkyne compound and maintaining the storage stability of the composition containing the halogenated alkyne compound, during the transportation of the composition containing the halogenated alkyne compound in a container such as a cylinder.

The composition containing a halogenated alkyne compound of the present disclosure contains at least one component selected from the group consisting of a halogenated ethylene compound, oxygen, water, hydrogen fluoride, and hydrogen chloride, has improved storage stability, and suppresses the polymerization or decomposition of the halogenated alkyne compound even under severe conditions during marine transportation in containers (e.g. temperature rise during transportation); thus, it is possible to stably transport the composition containing the halogenated alkyne compound.

## Claims

1. A composition comprising a halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
(2-1) a halogenated ethylene compound represented by formula (2): CF₂=CFX, wherein X represents a hydrogen atom, a chlorine atom, a bromine atom, or an iodine atom,
(2-2) oxygen,
(2-3) water,
(2-4) hydrogen fluoride, and
(2-5) hydrogen chloride,
wherein
in (2), based on the total amount of the composition,
when the composition contains (2-1) the halogenated ethylene compound represented by formula (2), the content of the halogenated ethylene compound represented by formula (2) is 1 vol ppm or more and 100 vol ppm or less,
when the composition contains (2-2) the oxygen, the content of the oxygen is 0.1 vol ppm or more and 100 vol ppm or less,
when the composition contains (2-3) the water, the content of the water is 0.1 vol ppm or more and 200 vol ppm or less,
when the composition contains (2-4) the hydrogen fluoride, the content of the hydrogen fluoride is 0.1 vol ppm or more and 10 vol ppm or less, or
when the composition contains (2-5) the hydrogen chloride, the content of the hydrogen chloride is 0.1 vol ppm or more and 10 vol ppm or less.

2. The composition according to claim 1, wherein the content of (1) the halogenated alkyne compound represented by formula (1) is 95 vol% to 99.9999 vol% based on the total amount of the composition.

3. A method for storing a composition containing a halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
(2-1) a halogenated ethylene compound represented by formula (2): CF₂=CFX, wherein X represents a hydrogen atom, a chlorine atom, a bromine atom, or an iodine atom,
(2-2) oxygen,
(2-3) water,
(2-4) hydrogen fluoride, and
(2-5) hydrogen chloride,
the method comprising, in (2), based on the total amount of the composition,
adjusting the content of (2-1) the halogenated ethylene compound represented by formula (2) to 1 vol ppm or more and 100 vol ppm or less when the composition contains the halogenated ethylene compound represented by formula (2),
adjusting the content of (2-2) the oxygen to 0.1 vol ppm or more and 100 vol ppm or less when the composition contains the oxygen,
adjusting the content of (2-3) the water to 0.1 vol ppm or more and 200 vol ppm or less when the composition contains the water,
adjusting the content of (2-4) the hydrogen fluoride to 0.1 vol ppm or more and 10 vol ppm or less when the composition contains the hydrogen fluoride, or
adjusting the content of (2-5) the hydrogen chloride to 0.1 vol ppm or more and 10 vol ppm or less when the composition contains the hydrogen chloride.

4. The method according to claim 3, comprising adjusting the content of (1) the halogenated alkyne compound represented by formula (1) to 95 vol% to 99.9999 vol% based on the total amount of the composition.

5. A method for suppressing a decrease in purity of a halogenated alkyne compound in a composition containing the halogenated alkyne compound, the composition comprising
(1) a halogenated alkyne compound represented by formula (1): R¹-C≡C-R², wherein R¹ represents a perfluoroalkyl group, and R² represents a perfluoroalkyl group, a hydrogen atom, or a fluorine atom, and
(2) at least one component selected from the group consisting of
(2-1) a halogenated ethylene compound represented by formula (2): CF₂=CFX, wherein X represents a hydrogen atom, a chlorine atom, a bromine atom, or an iodine atom,
(2-2) oxygen,
(2-3) water,
(2-4) hydrogen fluoride, and
(2-5) hydrogen chloride,
the method comprising, in (2), based on the total amount of the composition,
adjusting the content of (2-1) the halogenated ethylene compound represented by formula (2) to 1 vol ppm or more and 100 vol ppm or less when the composition contains the halogenated ethylene compound represented by formula (2),
adjusting the content of (2-2) the oxygen to 0.1 vol ppm or more and 100 vol ppm or less when the composition contains the oxygen,
adjusting the content of (2-3) the water to 0.1 vol ppm or more and 200 vol ppm or less when the composition contains the water,
adjusting the content of (2-4) the hydrogen fluoride to 0.1 vol ppm or more and 10 vol ppm or less when the composition contains the hydrogen fluoride, or
adjusting the content of (2-5) the hydrogen chloride to 0.1 vol ppm or more and 10 vol ppm or less when the composition contains the hydrogen chloride.

6. The method according to claim 5, comprising adjusting the content of (1) the halogenated alkyne compound represented by formula (1) to 95 vol% to 99.9999 vol% based on the total amount of the composition.
